# EUROPÄISCHE PATENTSCHRIFT

(11) **EP 2 231 049 B1**
(45) Veröffentlichungstag und Bekanntmachung des Hinweises auf die Patenterteilung: **24.06.2015**
(21) Anmeldenummer: 08861459.9
(22) Anmeldetag: 12.12.2008
(51) Int. Cl.: A61B 18/14

(54) **NEUTRALELEKTRODENERKENNUNG**
NEUTRAL ELECTRODE DETECTION
IDENTIFICATION D'ÉLECTRODES NEUTRES

(30) Priorität: 14.12.2007 DE 102007060431
(43) Veröffentlichungstag der Anmeldung: 29.09.2010
(73) Patentinhaber: ERBE Elektromedizin GmbH, 72072 Tübingen (DE)
(72) Erfinder: REICK, Michael, 73061 Ebersbach (DE)
(74) Vertreter: Bohnenberger, Johannes
(86) Internationale Anmeldenummer: PCT/EP2008/010590
(87) Internationale Veröffentlichungsnummer: WO 2009/077132

(56) Entgegenhaltungen:
- WO-A-97/24155
- US-A- 6 115 638
- US-A1- 2003 055 478
- US-B1- 6 217 574
- US-B1- 7 258 688

## Beschreibung

Die Erfindung betrifft ein elektrochirurgisches Gerät nach dem Oberbegriff des Patentanspruches 1, wie schon aus US7258688 bekannt, sowie eine Verwendung einer Kunststoff- oder Keramikfolie nach Anspruch 3 und ein Verfahren zur Herstellung und Inbetriebnahme einer Neutralelektrode nach Anspruch 4.

Bei elektrochirurgischen Geräten ist es bekannt, an ein- und dasselbe Gerät verschiedene Instrumente anzuschließen. Um das elektrochirurgische Gerät nun auf das angeschlossene Instrument einzustellen, ist es beispielsweise aus der DE 43 39 049 C2 bekannt, eine Codierung vorzunehmen, die beispielsweise über einen im Instrument vorhandenen Widerstand und dessen elektrischen Anschluss an das elektrochirurgische Gerät diesem mitgeteilt wird, so dass aus einer Vergleichsliste die Daten des angeschlossenen Instrumentes ausgelesen und das elektrochirurgische Gerät dementsprechend eingestellt werden kann.

In vielen Fällen werden monopolare Instrumente in der Elektrochirurgie verwendet, bei welchen auf den Hautabschnitt eines Patienten eine Neutralelektrode aufgeklebt wird. Derartige Neutralelektroden sind oftmals mit zwei oder mehr Wirkflächen ausgestattet, um einen korrekten Sitz der Neutralelektrode auf dem Hautabschnitt des Patienten feststellen zu können. Derartige Neutralelektroden werden in verschiedensten Ausführungen geliefert, je nachdem, welche Flächen oder Flächengrößen benötigt werden. Es ist leicht vorstellbar, dass in der Neonatal-Chirurgie andere Neutralelektroden verwendet werden müssen als bei Operationen an einem erwachsenen Patienten. Dies bedeutet, dass je nach verwendeter Neutralelektrode das elektrochirurgische Gerät zumindest teilweise auf andere Parameter eingestellt werden muss. Dies ist nicht nur arbeitsaufwändig, es können vielmehr auch Fehler auftreten, die besonders in diesem Gebiet der Technik fatale Folgen haben können.

Der Erfindung liegt die Aufgabe zu Grunde, ein elektrochirurgisches Gerät bzw. ein Verfahren zur Herstellung und Inbetriebnahme einer Neutralelektrode dahin gehend aufzuzeigen, dass bei vereinfachter Bedienbarkeit eine erhöhte Sicherheit gewährleistet ist.

Diese Aufgabe wird durch ein elektrochirurgisches Instrument nach Anspruch 1 sowie ein Verfahren nach Anspruch 4 gelöst. Eine besondere Verwendung ist in Anspruch 3 erläutert.

Ein wesentlicher Punkt der Erfindung liegt darin, dass ein ohnehin notwendiges Bauteil, nämlich eine Abdeckfolie, die immer Verwendung findet, um eine Neutralelektrode verpacken, sterilisieren und in sterilem Zustand auf die Hautoberfläche eines Patienten aufkleben zu können, als Codierungseinrichtung verwendet, die nach der Codierung des elektrochirurgischen Gerätes weggeworfen werden kann. Dadurch ist sowohl der Fertigungsaufwand einer Neutralelektrode mit einer Codierungseinrichtung sehr gering, andererseits wird die Verwendung auch erheblich erleichtert. Damit wiederum wird eine erhöhte Sicherheit geschaffen.

Man kann die Abdeckfolie mit leitenden Abschnitten (mit geringem Widerstand) ausstatten, die mit den Elektroden im abgedeckten Zustand derselben in Kontakt stehen und zwischen diesen definierte Widerstände z.B. als gesonderte Bauteile vorsehen. Einfacher ist es, als Abdeckfolie ein Material zu verwenden, das einen definierten spezifischen Widerstand aufweist. Dies wird dann besonders einfach und führt zu gut reproduzierbaren Ergebnissen, wenn die Abdeckfolie ein homogenes Material ist, dessen elektrische Parameter bei der Fertigung festgelegt werden.

Das elektrochirurgische Gerät weist eine Widerstandsmesseinrichtung auf, mittels derer der Widerstand zwischen den elektrisch isolierten Elektroden gemessen wird, der wiederum durch die aufgeklebte Abdeckfolie definiert ist.

Im einfachsten Fall kann der gemessene Widerstand angezeigt und abgelesen werden, so dass eine Bedienungsperson anhand des gemessenen Widerstands die verwendete Neutralelektrode identifiziert und das elektrochirurgische Gerät entsprechend einstellt. Vereinfacht wird die Bedienung dann, wenn eine Decodiereinrichtung vorgesehen ist, welche den gemessenen Widerstand mit gespeicherten Widerstandswerten vergleicht und aus dem Vergleichsergebnis direkt den verwendeten Typ der Neutralelektrode anzeigt. Eine weitere Vereinfachung der Bedienung ergibt sich dann, wenn aus dem Vergleichsergebnis bzw. aus dem gemessenen Widerstand direkt diejenigen Parameter abgeleitet und dem elektrochirurgischen Gerät zugeführt werden, die der angeschlossenen Neutralelektrode entsprechen.

Ein wesentlicher Parameter, der vorzugsweise mittels der beschriebenen Einrichtung im elektrochirurgischen Gerät eingestellt wird, ist der Maximalstrom, der über die Neutralelektrode und das verwendete elektrochirurgische Gerät durch den Patienten fließt. So wird beispielsweise der Maximalstrom auf etwa 300 mA begrenzt, wenn eine Neutralelektrode für eine Operation an Neugeborenen (Neonatal-Chirurgie) vorgesehen ist. Es muss also in diesem Fall eine Strombegrenzung nicht mehr durch das Operationspersonal eingestellt werden, es erfolgt diese Einstellung vielmehr automatisch aufgrund der gemessenen Widerstandswerte

Aus Obigem geht hervor, dass die Erfindung auch die Verwendung einer an sich bekannten Kunststoff- oder Keramikfolie mit einem definierten Widerstand betrifft, nämlich die Verwendung als Abdeckfolie einer Neutralelektrode zum Abdecken deren Wirkflächen.

Das hier vorgestellte Verfahren betrifft also die Herstellung und die Inbetriebnahme einer Neutralelektrode, wobei das Verfahren folgende Schritte umfasst:
a) Es wird eine Neutralelektrode in an sich bekannter Weise hergestellt, die mindestens zwei voneinander elektrisch isolierte Elektroden aufweist, welche Wirkflächen bilden, zum Aufbringen auf einen Hautabschnitt eines Patienten.
b) Auf diese Wirkflächen wird eine Abdeckfolie mittels einer Klebe- oder Adhäsions-Haftschicht aufgebracht, so dass sie während der Handhabung der Neutralelektrode (bei Verpackung und Versand) haften bleibt, vor einer Operation aber abgezogen werden kann. Die Abdeckfolie weist einen definierten elektrischen Widerstand derart auf, dass die Wirkflächen der Neutralelektrode über diesen Widerstand miteinander verbunden sind. Die so aufgebaute und geschützte Neutralelektrode kann nun verpackt und sterilisiert werden.
c) Nach dem Herausnehmen aus der Verpackung wird die Neutralelektrode mit noch darauf befindlicher Abdeckfolie an einen elektrochirurgischen Generator angeschlossen.
d) Mittels einer Messeinrichtung wird der Widerstand zwischen den Elektroden festgestellt.
e) Nunmehr wird der Generator entsprechend dem festgestellten Widerstand eingestellt, da dieser Widerstand charakteristisch ist für die angeschlossenen Neutralelektrode.
f) Zum Schluss wird die Abdeckfolie abgezogen, so dass die Neutralelektrode auf den dafür vorgesehenen Hautabschnitt eines Patienten aufgebracht werden kann.

Vorzugsweise wird der Generator entsprechend dem gemessenen Widerstandswert also entsprechend dem gemessenen Neutralelektrodentyp auf einen Maximalstrom eingestellt. Dies ist dann besonders einfach, wenn der gemessene Widerstandswert mit gespeicherten Werten verglichen wird, die jeweils einen bestimmten Neutralelektrodentyp repräsentieren.

Nachfolgend wird ein Ausführungsbeispiel der Erfindung anhand von Abbildungen näher erläutert. Hierbei zeigen
- Fig. 1 eine Draufsicht auf eine Ausführungsform der Neutralelektrode,
- Fig. 2 einen Schnitt entlang der Linie II-II aus Fig. 1,
- Fig. 3 eine Darstellung ähnlich der nach Fig. 2, jedoch mit abgenommener Abdeckfolie und in aufgeklebtem Zustand und
- Fig. 4 eine schematisierte Darstellung einer Ausführungsform des erfindungsgemäßen elektrochirurgischen Gerätes in Kombination mit der Neutralelektrode.

In der nachfolgenden Beschreibung werden für gleiche und gleich wirkende Teile dieselben Bezugsziffern verwendet.

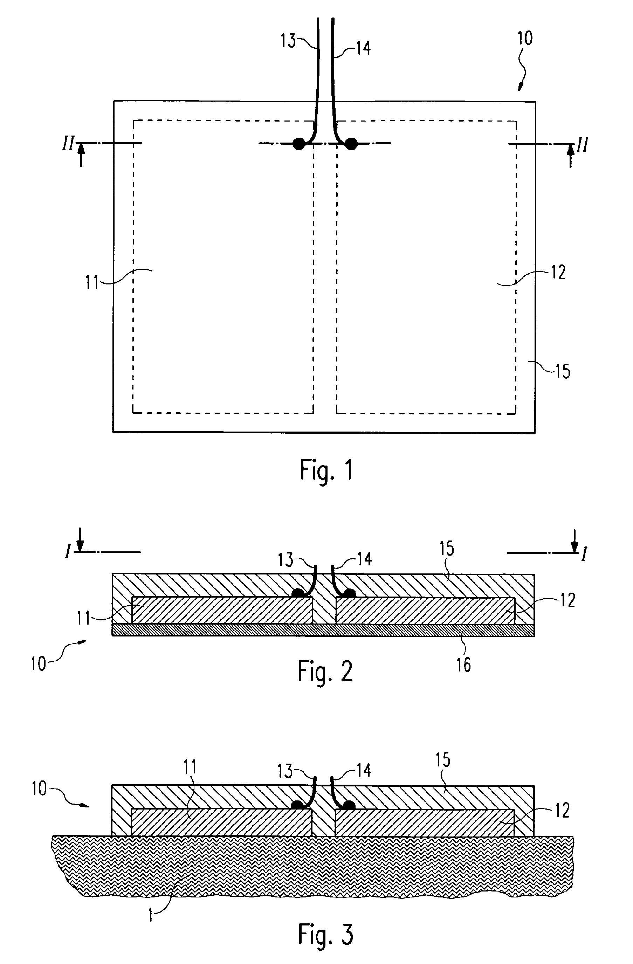

Wie aus den Fig. 1 und 2 hervorgeht, umfasst die Neutralelektrode zwei meist metallische Elektroden 11, 12, die auf einen Träger 15 aufgebracht und mit diesem fest verbunden sind. Wirkflächen der Elektroden 11, 12, die nicht mit dem Träger verbunden bzw. von diesem abgedeckt sind, sind in dem in Fig. 2 gezeigten Zustand der Elektrode, also vor deren Verwendung, durch eine Abdeckfolie 16 abgedeckt. Die Abdeckfolie 16 kann, wie in Fig. 2 angedeutet, aus einem homogenen Material gefertigt sein, das einen definierten elektrischen Widerstand aufweist. Es ist aber auch möglich, gesonderte Kontakte und einen dazwischen liegenden Widerstand (der wiederum als Folie ausgebildet sein kann) zu verwenden, um zwischen den Elektroden 13 und 14 den genannten definierten Widerstand zu bilden.

Zum Anschluss an ein elektrochirurgisches Gerät sind Anschlussleitungen 13, 14 vorgesehen, die mit den Elektroden 11, 12 elektrisch leitend verbunden sind.

Zum Aufbringen auf einen Hautabschnitt 1 eines Patienten wird die Abdeckfolie 16, die mit den Elektroden 11, 12 und Teil des Trägers 15 durch eine Klebe- oder Haftschicht verbunden ist, abgezogen, worauf hin die Neutralelektrode 10 auf den Hautabschnitt 1 des Patienten aufgeklebt oder auf andere Weise mit diesem Hautabschnitt verbunden werden kann. Gewöhnlich findet hier auch ein leitfähiges Gel Verwendung, um den Widerstand zwischen den Elektroden 11 und 12 und dem Hautabschnitt 1 zu verringern. Dies ist aber an sich bekannt.

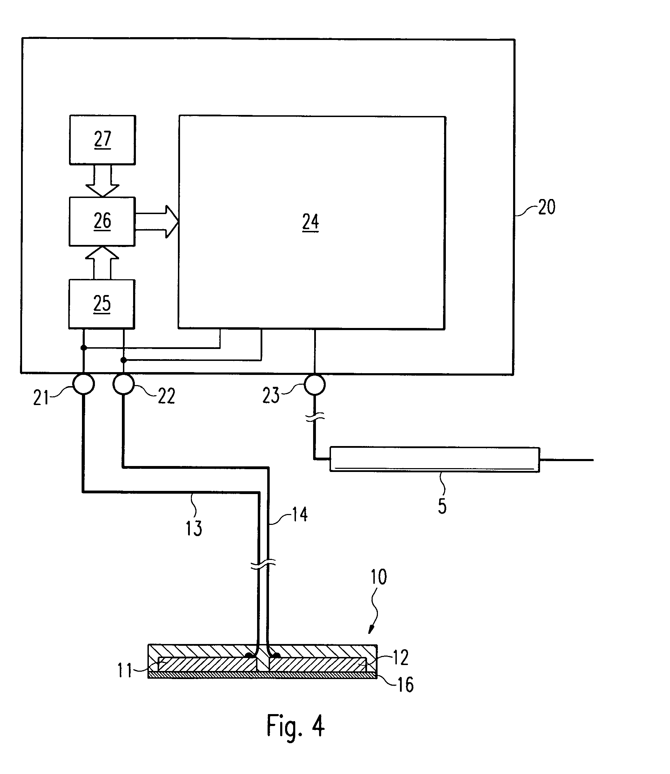

Das elektrochirurgische Gerät ist in Fig. 4 schematisiert dargestellt und mit der Bezugsziffer 20 versehen.

Das elektrochirurgische Gerät 20 weist Anschlussklemmen 21, 22 für die Neutralelektrode 10 und eine (oder mehrere) Klemme 23 zum Anschluss eines elektrochirurgischen Gerätes 5 auf. Diese Klemmen 21-23 sind mit einem Hochfrequenzgenerator 24 verbunden.

Im elektrochirurgischen Gerät 20 ist weiterhin eine Widerstandsmesseinrichtung 25 vorgesehen, welche mit den Anschlussklemmen 21 und 22 und über die Anschlussleitungen 13, 14 mit den Elektroden 11, 12 der Neutralelektrode 10 verbunden sind. Die Widerstandsmesseinrichtung (ggf. Impedanzmesseinrichtung) stellt über einen Messstrom, der von einer der Elektroden 11 über die elektrisch leitende Abdeckfolie 16 zur anderen Elektrode 12 fließt, fest, wie groß der elektrische Widerstand zwischen den beiden Elektroden 11, 12 ist. Der festgestellte Widerstandswert wird von der Widerstandsmesseinrichtung 25 einem Vergleicher 26 zugeführt, der den gemessenen Wert mit Werten vergleicht, die in einem Speicher 27 vorliegen. Die gespeicherten Werte entsprechen verschiedenen Ausführungsformen von Neutralelektroden 10. Das Vergleichsergebnis wird vom Vergleicher 26 dem HF-Generator 24 übermittelt, der daraufhin Arbeitsparameter einstellt. Ein solcher Arbeitsparameter ist insbesondere ein Maximalstrom, der vom HF-Generator abgegeben werden kann, was insbesondere bei Neonatal-Operationen von höchster Wichtigkeit ist.

### Bezugszeichenliste

- 1: Hautabschnitt
- 2: elektrochirurgisches Instrument
- 10: Neutralelektrode
- 11, 12: Elektrode
- 13, 14: Anschlussleitung
- 15: Träger
- 16: Abdeckfolie
- 20: elektrochirurgischer Generator
- 21, 22, 23: Anschlussklemme
- 24: HF-Generator
- 25: Widerstandsmesseinrichtung
- 26: Vergleicher
- 27: Speicher

## Patentansprüche

1. Elektrochirurgisches Gerät zum Anschluss einer Neutralelektrode (10),
die mindestens zwei voneinander elektrisch isolierte Elektroden (11, 12) aufweist, wobei auf Wirkflächen der Elektroden (11, 12), die auf einen Hautabschnitt (1) eines Patienten aufbringbar sind, eine Abdeckfolie (16) befestigt und vor dem Aufbringen abziehbar ist,
wobei die Abdeckfolie (16) einen definierten Widerstand aufweist, sodass zwischen den Elektroden (11,12) ein definierter Widerstand gebildet ist,
**dadurch gekennzeichnet, dass** das elektrochirurgische Gerät umfasst:
- eine Widerstandsmesseinrichtung (25) zum Messen eines Widerstands zwischen den Wirkflächen der Elektroden (11, 12) der Neutralelektrode, wenn die Abdeckfolie (16) angebracht ist,
- eine(n) Decodiereinrichtung/Vergleicher (26) zum Vergleichen des gemessenen Widerstands mit gespeicherten Widerstandswerten und zum Bestimmen des Elektrodentyps der angeschlossenen Neutralelektrode anhand des gemessenen Widerstands, und
- eine Anzeige- und/oder Registriereinrichtung (24) zum Anzeigen und/oder Registrieren des Elektrodentyps, der dem Widerstandswert zugeordnet ist.

2. Elektrochirurgisches Gerät nach Anspruch 1,
**gekennzeichnet durch**
eine Strombegrenzungseinrichtung (24), die beim Vorliegen bestimmter Widerstandswerte oder Elektrodentypen einen vom elektrochirurgischen Gerät maximal abgebbaren Stromwert einstellt.

3. Verwendung einer Kunststoff- oder Keramikfolie mit definiertem Widerstand als Abdeckfolie der Neutralelektrode nach Anspruch 1 zum Codieren des Elektrodentyps der Neutralelektrode.

4. Verfahren zur Herstellung und Inbetriebnahme einer Neutralelektrode, umfassend die Schritte
- Herstellen einer Neutralelektrode mit mindestens zwei voneinander elektrisch isolierten Elektroden, die Wirkflächen zum Aufbringen auf einem Hautabschnitt eines Patienten aufweisen;
- Aufbringen einer Abdeckfolie, die einen definierten elektrischen Widerstand aufweist, auf die Wirkabschnitte derart, dass diese über die Abdeckfolie miteinander elektrisch verbunden sind;
- Anschließen der Neutralelektrode an einen elektrochirurgischen Generator;
- Feststellen des Widerstands zwischen den Elektroden durch eine Widerstandsmesseinrichtung (25);
- Vergleichen des festgestellten Widerstands mit gespeicherten Werten;
- Auswahl eines Elektrodentyps der Neutralelektrode anhand des festgestellten Widerstands;
- Einstellen des Generators zum Betreiben des Elektrodentyps der Neutralelektrode und
- Entfernen der Abdeckfolie und Aufbringen der Neutralelektrode auf den Hautabschnitt.

5. Verfahren nach Anspruch 4,
**dadurch gekennzeichnet, dass**
der Generator auf einen Maximalstrom eingestellt wird.

## Claims

1. Electrosurgical appliance for connecting a neutral electrode (10)
that has at least two electrodes (11, 12) electrically insulated from one another, wherein a cover foil (16) is secured to effective areas of the electrodes (11, 12) that can be applied to a skin section (1) of a patient, and can be removed prior to application, wherein the cover foil (16) has a defined resistance, so that a defined resistance is formed between the electrodes (11, 12),
**characterized in that** the electrosurgical appliance comprises:
- a resistance measuring device (25) for measuring a resistance between the effective areas of the electrodes (11, 12) of the neutral electrode when the cover foil (16) is attached,
- a decoding device/comparator (26) for comparing the measured resistance with stored resistance values and for determining the electrode type of the connected neutral electrode on the basis of the measured resistance, and
- a display and/or registration device (24) for displaying and/or registering the electrode type that is associated with the resistance value.

2. Electrosurgical appliance according to Claim 1,
**characterized by**
a current limiting device (24) that, when particular resistance values or electrode types are present, sets a maximum current value that can be output by the electrosurgical appliance.

3. Use of a plastic or ceramic foil having a defined resistance as the cover foil of the neutral electrode according to Claim 1 for coding the electrode type of the neutral electrode.

4. Method for producing and starting up a neutral electrode, comprising the steps of
- producing a neutral electrode having at least two electrodes electrically insulated from one another that have effective areas for application to a skin section of a patient;
- applying a cover foil that has a defined electrical resistance to the effective sections such that the latter are electrically connected to one another via the cover foil;
- connecting the neutral electrode to an electro-surgical generator;
- establishing the resistance between the electrodes using a resistance measuring device (25);
- comparing the established resistance with stored values;
- selecting an electrode type for the neutral electrode on the basis of the established resistance;
- setting the generator for operation of the electrode type of the neutral electrode, and
- removing the cover foil and applying the neutral electrode to the skin section.

5. Method according to Claim 4,
**characterized in that**
the generator is set to a maximum current.

## Revendications

1. Appareil électrochirurgical destiné à être raccordé à une électrode neutre (10), comprenant au moins deux électrodes (11, 12) électriquement isolées l'une de l'autre, dans lequel une feuille de protection (16) est fixée sur des surfaces actives des électrodes (11, 12), les surfaces actives pouvant être mises en place sur une section de la peau (1) d'un patient, et peut en être retirée avant la mise en place,
dans lequel la feuille de protection (16) présente une résistance définie, de manière à ce qu'une résistance définie soit établie entre les électrodes (11, 12), **caractérisé en ce que** l'appareil électrochirurgical comprend :
- un dispositif de mesure de résistance (25) destiné à mesurer une résistance entre les surfaces actives des électrodes (11, 12) de l'électrode neutre lors de la mise en place de la feuille de protection (16),
- un dispositif de décodage/comparateur (26) destiné à comparer la résistance mesurée à des valeurs de résistance stockées et à déterminer le type d'électrode de l'électrode neutre raccordée sur la base de la résistance mesurée, et
- un dispositif d'affichage et/ou d'enregistrement (24) destiné à afficher et/ou à enregistrer le type d'électrode qui est associé à la valeur de résistance.

2. Appareil électrochirurgical selon la revendication 1,
**caractérisé par** un dispositif limiteur de courant (24) qui règle une valeur de courant maximale pouvant être délivrée par l'appareil électrochirurgical pour des valeurs données de la résistance ou des types donnés d'électrodes.

3. Utilisation d'une feuille de matière plastique ou de céramique ayant une résistance définie en tant que feuille de protection de l'électrode neutre selon la revendication 1 pour coder le type d'électrode de l'électrode neutre.

4. Procédé de fabrication et de mise en fonctionnement d'une électrode neutre, comprenant les étapes consistant à
- réaliser une électrode neutre comportant au moins deux électrodes électriquement isolées l'une de l'autre, qui présentent des surfaces actives devant être mises en place sur une section de la peau d'un patient ;
- mettre en place une feuille de protection qui présente une résistance électrique définie sur les sections actives, de manière à ce qu'elles soient électriquement reliées les unes aux autres par l'intermédiaire de la feuille de protection ;
- raccorder l'électrode neutre à un générateur électrochirurgical ;
- déterminer la résistance entre les électrodes au moyen d'un dispositif de mesure de résistance (25) ;
- comparer la résistance déterminée à des valeurs stockées ;
- sélectionner un type d'électrode de l'électrode neutre sur la base de la résistance déterminée ;
- régler le générateur pour la mise en fonctionnement du type d'électrode de l'électrode neutre et
- enlever la feuille de protection et mettre en place l'électrode neutre sur la section de la peau.

5. Procédé selon la revendication 4,
**caractérisé en ce que** le générateur est réglé sur un courant maximal.
